# FASCICULE DE BREVET EUROPEEN

(11) **EP 0 961 598 B1**
(45) Date de publication et mention de la délivrance du brevet: **08.09.2004**
(21) Numéro de dépôt: 98946527.3
(22) Date de dépôt: 29.09.1998
(51) Int. Cl.: A61F 2/06, A61B 17/068

(54) **ENSEMBLE MEDICALE POUR LE TRAITEMENT D'UNE AFFECTION DE CONDUIT ANATOMIQUE**
MEDIZINISCHE VORRICHTUNG ZUR BEHANDLUNG EINER BESCHÄDIGUNG EINER ANATOMISCHEN LEITUNG
MEDICAL DEVICE FOR THE TREATMENT OF A DISEASED ANATOMICAL DUCT

(30) Priorité: 16.12.1997 FR 9715969; 29.12.1997 FR 9716625
(43) Date de publication de la demande: 08.12.1999
(73) Titulaire: B. Braun Celsa, 92100 Boulogne-Billancourt (FR)
(72) Inventeur: CHEVILLON, Gérard, F-92100 Montrouge (FR); NADAL, Guy, F-86000 Poitiers (FR); ANIDJAR, Samy, F-75013 Paris (FR)
(74) Mandataire: Lerner, François
(86) Numéro de dépôt international: PCT/FR1998/002086
(87) Numéro de publication internationale: WO 1999/030637

(56) Documents cités:
- EP-A- 0 541 987
- EP-A- 0 688 544
- WO-A-97/39687
- FR-A- 2 746 292
- GB-A- 2 269 104

## Description

L'invention concerne un ensemble médical améliorant les conditions de mise en place et de tenue d'un implant dans un conduit anatomique, en particulier s'il s'agit d'implant, ou d'endoprothèse, vasculaire pour anévrisme adapté(e) pour être mis(e) en place au moins en partie par voie endoluminale percutanée.

Dans le domaine, on connaît différents types d'implants et différentes techniques de pose.

Ainsi, aux brevets US-A-5 527 355 ou US-A-5 707 378, est-il enseigné un appareil et une méthode de traitement d'anévrisme par application d'une bande autour de la surface extérieure du vaisseau, aux endroits où sont situés les moyens-ressort de maintien de l'implant contre le vaisseau (stents).

Ainsi, est-il connu un ensemble médical pour le traitement d'une affection d'un conduit anatomique comprenant :
- un implant introductible dans le conduit à traiter, l'implant ayant un axe général et présentant sensiblement selon cet axe deux extrémités libres, respectivement proximale et distale,
- au moins une doublure pour doubler extérieurement une partie au moins de la paroi du conduit, sensiblement en regard de l'extrémité proximale de l'implant,
- et des moyens de fixation de la doublure à l'implant, à travers la paroi du conduit, pour appliquer intérieurement l'implant contre la paroi du conduit.

Dans ces brevets, il est même prévu deux bandes de doublure, aux extrémités proximale et distale de l'implant, lequel est adapté pour pouvoir être introduit dans un état radialement resserré. Après quoi, il occupe un état radialement déployé pour traiter l'anévrisme.

Un certain nombre de problèmes demeurent malgré tout liés aux conditions de mise en place de la bande autour du conduit considéré, de fixation de l'implant à la paroi de ce conduit, ainsi qu'aux possibles conditions d'étanchéité efficace à obtenir vis-à-vis du fluide pouvant y circuler.

A noter que dans ces brevets, le système d'attache de chaque bande autour du conduit peut poser un problème, en particulier s'il s'agit de l'aorte, dès lors qu'il peut être particulièrement délicat de chercher à en faire entièrement le tour.

Il peut être en outre difficile pour le praticien de réussir à positionner précisément la bande de doublure en face de l'extrémité correspondante de l'implant, dès lors qu'à défaut de cela, la fixation de l'un à l'autre risquera d'être défectueuse. En effet, cette fixation s'opère par les crochets liés à l'un des stents de l'implant.

Le document WO 97/39687 décrit un appareil et un procédé de réparation d'un anévrisme dans lesquels un collier extérieur est placé sous forme d'une sangle entourant les extrémités d'un implant, afin qu'un ressort de fixation muni de griffes soit retenu. La sangle a pour seul rôle de retenir la paroi extérieure d'un conduit anatomique qui est poussé de l'intérieur localement par des griffes. Elle joue un rôle passif d'arrêt des pointes des griffes.

Le document EP-A 0 541 987 décrit un appareil destiné à l'application endoscopique d'agrafes chirurgicales. Dans les différents modes de réalisation décrits, l'agrafe chirurgicale traverse une nappe chirurgicale (112) et un tissu biologique (114).

Aucun de ces documents ne décrit un système dans lequel une doublure extérieure contre laquelle vient s'appliquer une partie d'agrafe n'est armée afin qu'elle ne puisse pas être.traversée par l'agrafe lorsque celle-ci est fermement fixée à un organe placé à l'intérieur d'un conduit anatomique.

L'invention concerne un tel ensemble médical, tel que défini dans les revendications.

Une description encore plus détaillée de l'invention va maintenant être fournie en référence aux dessins annexés dans lesquels :
- la figure 1 montre l'implant en place dans un anévrisme aortique avec deux bandes de doublure prêtes à l'entourer,
- la figure 2 présente en perspective et en vue agrandie une réalisation possible d'une de ces bandes,
- la figure 3 est une variante de réalisation d'une bande selon la même vue que la figure 2,
- la figure 4 montre une aiguille avec fil de suture (sans prétention d'échelle),
- la figure 5 schématise en vue agrandie une agrafe de fixation, dans son état originel,
- la figure 6 schématise (à plus petite échelle) la mise en place d'une variante de réalisation de l'implant de la figure 1, avec une seule doublure,
- la figure 7 montre une étape d'implantation antérieure à la situation de la figure 6,
- la figure 8 montre une étape d'implantation intermédiaire entre les situations des figures 6 et 7,
- la figure 9 montre, en vue agrandie, une zone locale de fixation d'un implant, avec une agrafe serrée contre l'implant,
- la figure 10 montre une agrafe dans son état originel, engagée à travers un patin d'étanchéité,
- la figure 11 montre, en vue agrandie par rapport à la figure 1, une forme possible de réalisation d'une doublure de fixation,
- la figure 12 schématise à petite échelle l'opération de mise en place et d'agrafage des doublures, pour un implant tel qu'illustré sur la figure 1,
- et la figure 13 présente une vue locale d'une réalisation possible de doublure.

Dans ce qui suit, on ne considère que le cas d'un implant consistant en une endoprothèse destinée à traiter un anévrisme de l'aorte, même si d'autres vaisseaux, voire d'autres types d'affection, peuvent bénéficier des améliorations apportées par l'invention.

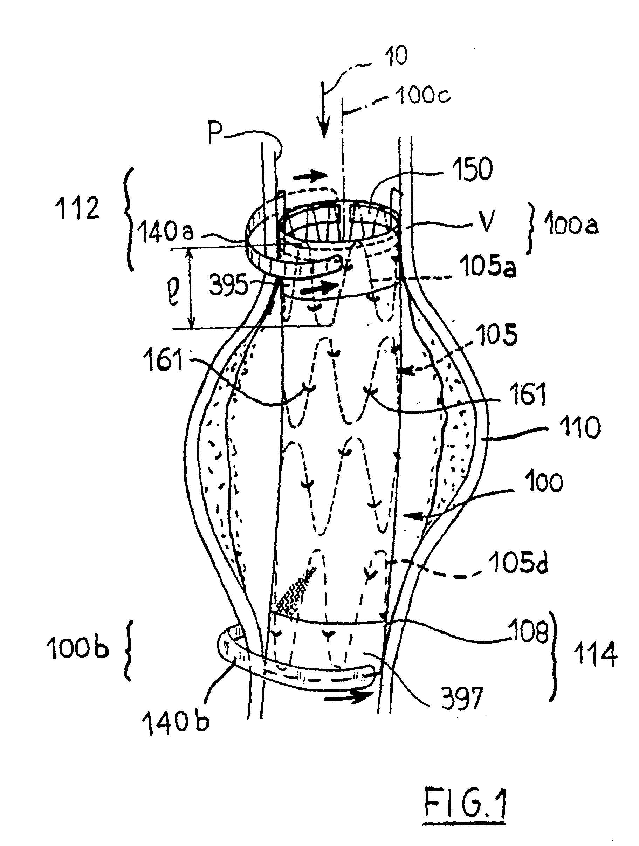

Ainsi, la figure 1 montre une aorte V atteinte d'un anévrisme 110. L'anévrisme est délimité par un collet supérieur (ou amont) 112 et un collet inférieur (ou aval) 114. Le repère 10 indique le sens de circulation du sang.

Une endoprothèse 100, d'axe 100c, est implantée au moins à l'endroit de l'anévrisme.

L'implant 100 peut en particulier se présenter comme décrit au brevet US-A-4 533 665, ou FR-A-2 732 404.

L'implant est plaqué, vers son extrémité proximale 100a, contre la paroi P du vaisseau V, par l'intermédiaire d'au moins un élargisseur radialement déployable, habituellement dénommé "stent", 105.

Outre le stent 105 (qui en l'espèce comprend quatre étages de fils métalliques en zigzags enroulés sur eux-mêmes à chaque étage), l'implant 100 comprend une gaine souple, synthétique, biocompatible, 108, par exemple en Dacron (marque déposée), permettant de canaliser le sang à travers elle, une fois la prothèse implantée. Chaque stent élémentaire est suturé à la gaine, en 161. On trouve un stent élémentaire vers les deux extrémités libres 100a, 100b de l'implant.

Le(chaque) stent 105 est en l'espèce radialement "autoexpansible" (comme dans FR-A-2 732 404), bien qu'il puisse, en variante, être constitué par un tube métallique ajouré, radialement déployable au-delà de sa limite élastique sous l'effet d'une force interne d'expansion radiale, tel qu'un ballon, comme dans US-A-4 533 665.

Etant donné qu'en l'espèce, le plaquage de l'implant contre les collets supérieur et inférieur s'effectue par l'intermédiaire des premier et dernier étages 105a, 105d de stent, ces étages pourront présenter une force radiale (ou une résistance à la compression radiale) supérieure à ce que présentent les autres étages et/ou le reste de l'implant. On obtient cet effet mécanique par exemple en augmentant le nombre de zigzags à ce premier étage et/ou en diminuant la longueur axiale ℓ de ces étages par rapport aux autres étages du stent. Une autre solution serait encore d'adjoindre un moyen de renfort rapporté, tel par exemple qu'une bague fendue 150.

Autour du conduit V, et plus particulièrement autour des collets supérieur et inférieur, apparaît une pièce 140a, 140b, de doublure du conduit.

Une fois en place, la bande va venir doubler localement la paroi du vaisseau pour favoriser la mise en place de moyens de fixation de chaque bande à l'implant 100, à travers donc la paroi du conduit V, afin d'appliquer intérieurement cet implant contre la paroi P, de préférence de façon étanche au sang.

Différentes réalisations des bandes peuvent être imaginées.

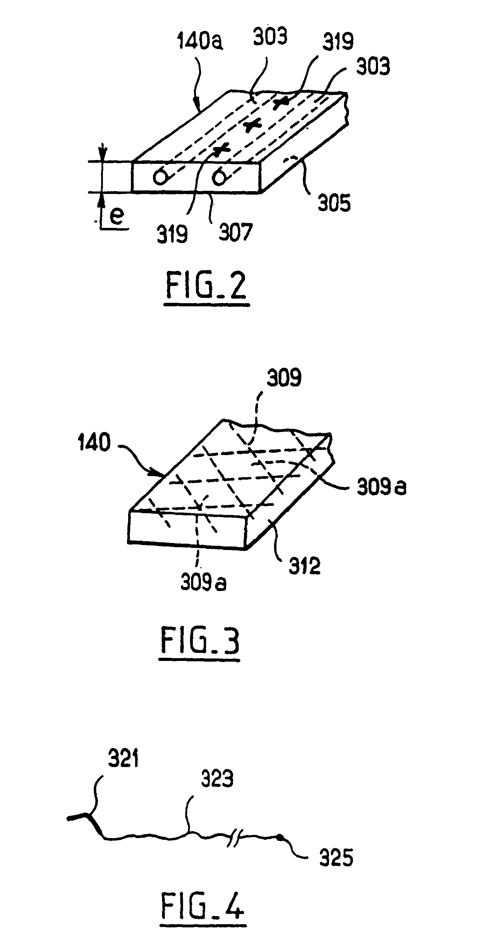

Si chaque bande est suffisamment résistante à l'écrasement, dans le sens de son épaisseur (repérée e sur la figure 2), la bande constituera alors avantageusement un étai extérieur pour les moyens de fixation, l'implant 100 pouvant, quant à lui, constituer l'étai interne, ceci par l'intermédiaire en l'espèce du stent 105, voire d'une bande, d'un patin local ou d'un anneau fendu complémentaire repéré(e) 150 sur la figure 1 et fixé(e) et rapporté(e) vers l'extrémité proximale de l'implant pour l'étayer.

Sur la figure 2, la doublure se présente comme une lanière renfermant un ou plusieurs fils (303) métalliques ou en matière plastique (en l'espèce, deux fils parallèles) noyés dans un tampon amortisseur 305 (par exemple, en feutre), le tout pouvant être recouvert d'une fine enveloppe 307, par exemple en Dacron (marque déposée).

Sur la figure 3, la bande 140a comprend un treillis relativement rigide 309, noyé dans une couche de protection 312 pour amortir le contact avec le vaisseau. Eventuellement, on pourrait ne prévoir la couche 312 que du côté intérieur où la bande vient au contact du vaisseau, l'armature d'étai 309 étant alors en surface.

Dans une autre variante non représentée, on pourrait imaginer de réaliser la bande comme un tampon de feutre renforcé par un entrelacs de filaments métalliques ou plastiques.

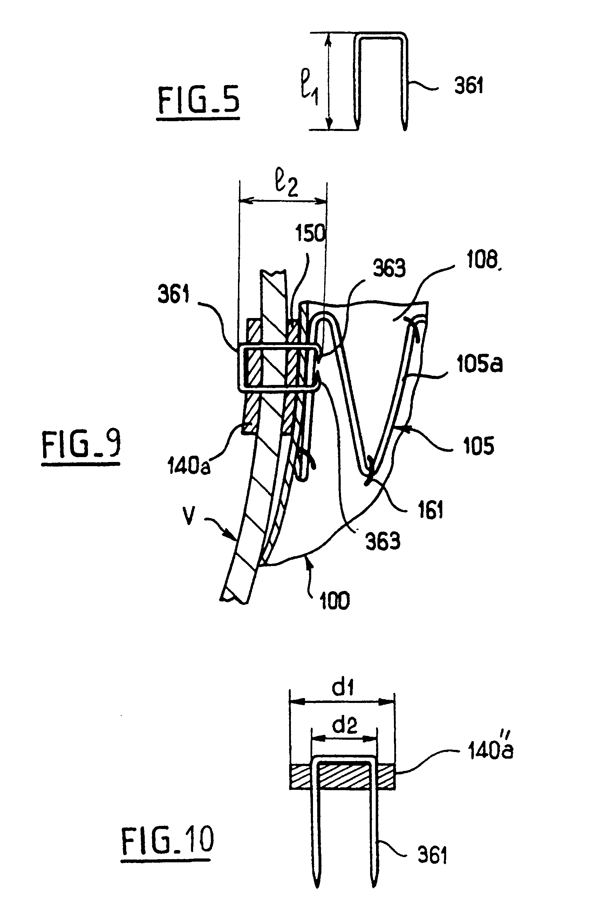

Concernant ces moyens de fixation, on notera que la solution de la figure 3, ou celle de feutre renforcé, sont en particulier appropriées pour une liaison par suture (fils chirurgicaux) entre la bande et l'implant 100, le praticien agissant alors depuis l'extérieur du vaisseau (par endoscopie ou après dénudation) tandis que la solution de la figure 2 peut être notamment prévue si le moyen de fixation est du type "agrafe", comme illustré en particulier sur les figures 5, 6 et 9, voire du type "rivet" comme dans FR 97 16625.

Si les moyens de fixation risquent d'exercer un effort de pénétration excessif sur la bande et/ou sur le manchon 108 de la prothèse, alors l'une et/ ou l'autre de ces deux pièces pourra présenter des passages ouverts tels que les découpes traversantes par exemple en croix ou en portion de cercle, comme figuré en 319 sur la figure 2. A noter que la taille des mailles 309a du treillis 309 de la figure 3 peut permettre d'obtenir le même effet.

Sur la figure 4, on a schématisé une aiguille de suture 321 courbe liée à son fil de suture 323 pour lier ensemble la prothèse et la bande de doublure, à travers la paroi du vaisseau. Le fil 323 est terminé par un noeud 325 permettant au fil de tenir en place après avoir été posé.

Sur la figure 5, une agrafe chirurgicale de fixation 361 en " 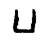 " a été schématisée.

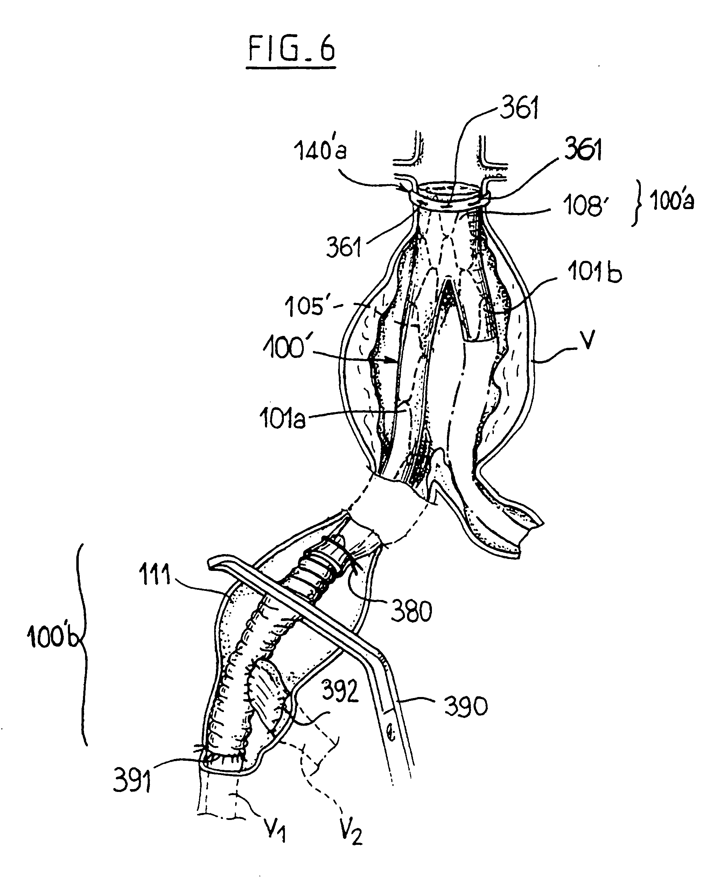

Sur la figure 6, on a représenté schématiquement une variante du cas de la figure 1. L'implant 100' est ici prévu pour être localement fixé et plaqué au conduit V par l'intermédiaire d'un seul collier ou lacet 140'a, à son extrémité proximale 100'a. L'extrémité distale 100'b est située à l'endroit du collet inférieur de l'anévrisme inférieur 111, près de l'embranchement du vaisseau iliaque externe V₁ et de l'artère hypogastrique V₂. L'implant 100' est dépourvu de crochet d'ancrage (alors qu'ils sont très souvent prévus dans l'art antérieur).

L'implant 100' comprend une partie proximale en forme de " 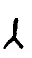 " avec un tronçon commun supérieur et deux branches inférieures 101a, 101b. La branche 101a est plus longue que l'autre qui se termine dans l'anévrisme aortique (avant d'être reliée ultérieurement à un implant de raccordement non décrit ici et figuré en traits fantômes).

L'implant 100' comprend une gaine en tissu 108' soutenue par une structure "en stents" étagés, présente à l'intérieur de la gaine sauf à la partie extrême distale 100'b où la gaine est, sur une certaine longueur (quelques centimètres) dépourvue de toute armature de soutien mécanique, comme décrit dans WO-A-97/41804 introduit dans la présente description, par référence.

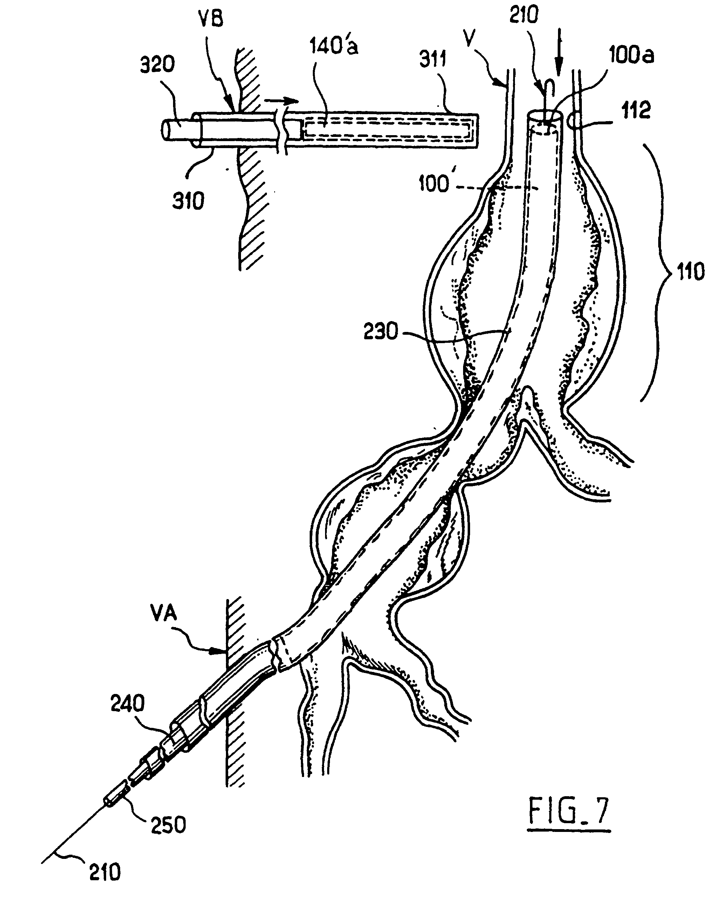

Sur la figure 7, on a représenté schématiquement la technique d'implantation de l'endoprothèse 100', par voie endoluminale percutanée (en particulier, technique dite de "SELDINGER"), ainsi que la mise en place du collier 140'a, par voie laparoscopique, de préférence.

Plus précisément, après avoir pratiqué une voie d'accès VA à travers la peau du patient, et avoir introduit un cathéter élargisseur (non représenté) sur le guide-fil 210, la gaine extérieure 230 renfermant une deuxième gaine 240 près de l'extrémité distale de laquelle a été préalablement préchargé l'implant 100', est introduite, après retrait de l'élargisseur.

Parvenu en amont dans la zone 110 de l'anévrisme aortique, l'extrémité proximale de l'implant est correctement positionnée, par voie radiographique.

Les deux gaines 230, 240, sont alors successivement retirées, le cathéter intérieur 250 servant, si nécessaire, d'appui arrière à l'implant, pour qu'il ne recule pas au moment du retrait des gaines.

L'implant 100' se déploie alors radialement de lui-même s'il est autoexpansible, ou sous l'effet d'un moyen de gonflage interne si un tel moyen a été prévu, ou encore par tout autre moyen équivalent.

Une fois l'implant déployé, une gaine 310 renfermant la doublure 140'a est engagée par voie laparoscopique (endoscopie), et ce sensiblement au niveau du collet amont 112 de l'anévrisme, transversalement à l'axe du vaisseau et donc de l'implant, via la voie d'accès percutanée VB pratiquée ici en zone abdominale.

Une fois l'extrémité distale 311 de la gaine 310 sensiblement en face de l'extrémité 100'a de l'implant, la gaine est retirée, avec utilisation à nouveau d'un cathéter d'appui arrière 320 sur lequel va pouvoir s'appuyer le collier 140'a qui, pour son introduction, aura pu être préalablement étendu à plat dans la gaine, au bout du "poussoir" 320.

Si la doublure 140'a est une bande présentant une capacité à se refermer sur elle-même en un anneau fendu, comme montré sur la figure 1, un largage progressif va lui permettre d'entourer naturellement le collet 112. Sinon, et en particulier si l'armature de la doublure est non élastiquement déformable, l'introduction (toujours par voie laparoscopique, de préférence à une dénudation toujours possible) d'une ou de plusieurs pinces doit permettre au praticien de conformer la bande 140'a à la forme extérieure locale de la zone 112, à la manière d'un anneau fendu. La déformabilité de la bande qui peut présenter une longueur comprise entre 2 et 6 cm environ et une épaisseur comprise entre 0,5 mm et quelques millimètres, facilite sa conformation.

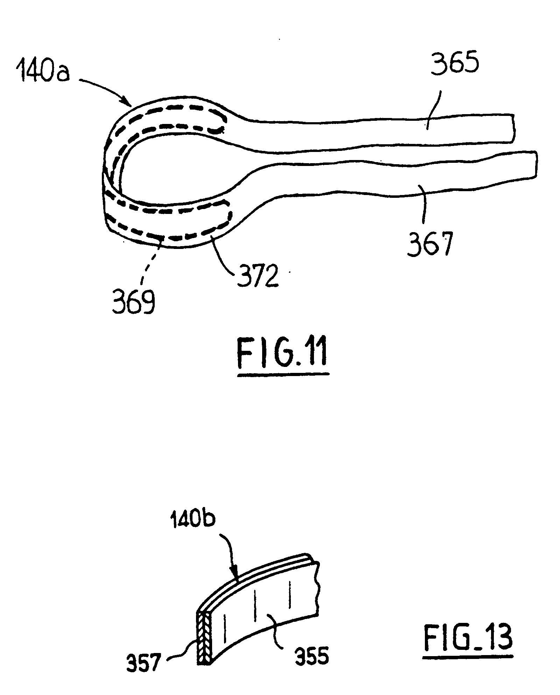

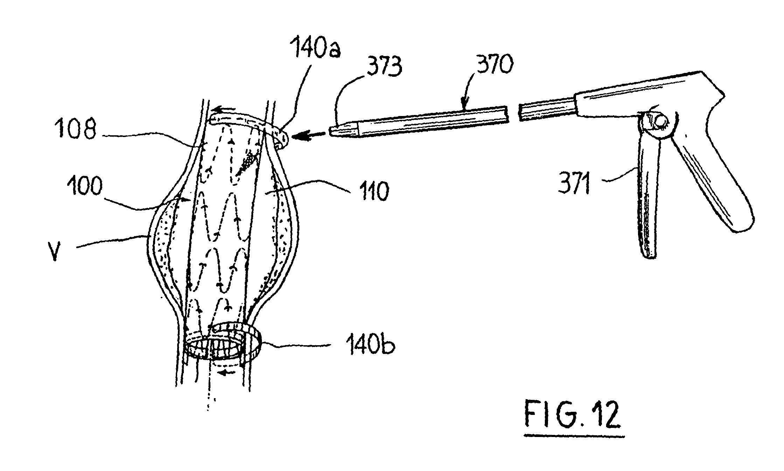

Si la doublure présente une surlongueur de sécurité (voir figure 11), une coupure (avec mise à longueur) est alors effectuée.

Si, pour la fixation de cette doublure, on utilise des moyens de suture (voir figure 4), alors une pince peut ensuite être utilisée pour manoeuvrer l'aiguille 321 (préalablement introduite également par voie laparoscopique à l'intérieur d'un cathéter ou d'une gaine introductrice), jusqu'à ce que le praticien ait pu passer le fil 323, plusieurs fois à travers la bande 140'a, la zone 112 du vaisseau et la zone 100a de l'implant, en faisant le tour d'une partie au moins du vaisseau. De préférence, le fil sera passé derrière le stent en regard et/ou à travers l'anneau d'étai de l'implant éventuellement prévu (repère 150 sur la figure 1), lequel pourrait être situé sur la périphérie extérieure de l'implant comme sur la figure 9. On pourrait aussi ne prévoir à sa place qu'un simple tampon, par exemple en feutre biocompatible revêtu extérieurement de silicone et fixé à l'endoprothèse.

Avantageusement, la suture sera serrée depuis l'extérieur du vaisseau, de manière à appliquer étroitement la portion 100'a de l'implant contre la surface intérieure du collet 112, assurant ainsi une étanchéité vis-à-vis du sang.

Il en sera de préférence de même si la fixation s'opère par l'intermédiaire d'agrafes telles que 361, comme le montre les figures 5, 8 et 9, la zone de pliure 363 des extrémités libres de chaque agrafe assurant un maintien efficace.

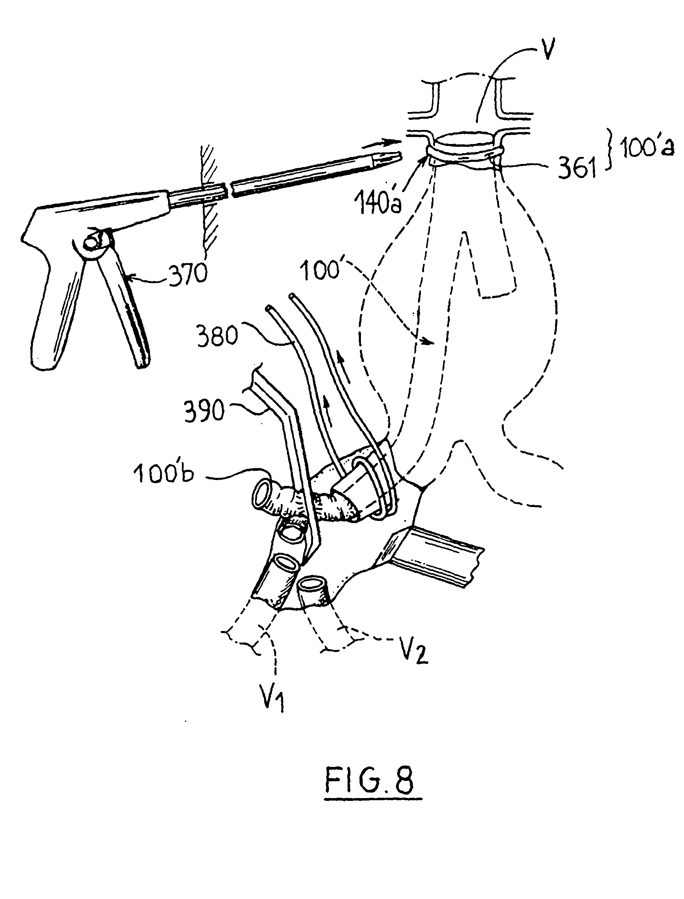

La figure 8 montre justement schématiquement comment pourrait être effectué l'agrafage de l'extrémité proximale 100'a de l'implant, avant que l'extrémité distale 100'b soit chirurgicalement fixée.

Pour l'agrafage, on décrira plus en détail ci-après le type d'agrafeuse et d'agrafe. On notera toutefois dès à présent la possibilité d'utiliser une agrafeuse 370 introduite par endoscopie jusqu'à la paroi extérieure du conduit et chargée avec des agrafes telles que 361 (figure 5), les agrafes étant déformées jusqu'à leur état de fixation en position serrée de la figure 9.

Une fois une série d'agrafes fixées sur le pourtour du collier 140'a, sensiblement perpendiculairement à l'axe du conduit, le praticien procède à la fixation de la zone avale 100'b de l'implant, comme décrit dans WO-A-97/41804. Brièvement, on rappelle qu'il a déjà dénudé les abords de l'anévrisme inférieur 111 et effectué les ligatures et pincements appropriés (tels que schématisés en 380, 390 sur la figure 8).

Il se saisit alors de l'extrémité 100'b non structurée qu'il coupe à longueur.

Il relie ensuite chirurgicalement cette extrémité coupée au(x) vaisseau(x) (par exemple par sutures, telles que 391, 392, figure 6).

Bien entendu, s'il y a deux vaisseaux à recanaliser (V₁, V₂), le bout 100'b est bifurqué comme sur les figures 6 et 8.

Pour mettre en place et maintenir l'éventuel implant de raccordement montré en traits fantômes sur la figure 6, le praticien peut ensuite procéder comme déjà enseigné dans l'art antérieur.

Après avoir vérifié que tous les instruments et moyens d'implantation ont été retirés, les voies d'abord sont ensuite refermées.

En tant que conduit autre que vasculaire sur lequel on pourrait intervenir dans le cadre de l'invention, on peut citer la vésicule biliaire (voir à cet égard WO-A-97/09008, pour les dimensions, matières, ..., cf. pages 8 à 11, en particulier).

A noter également qu'en alternative, l'endoprothèse 100 pourrait se présenter comme un tube bifurqué, comme dans US-A-5 527 355.

D'autres types d'implants endoluminaux pourraient également convenir.

Sur la figure 10, la doublure extérieure de conduit a été repérée 140"a. Il s'agit d'une pièce ronde (disque) ; mais elle peut avoir toute autre forme désirée (notamment rectangulaire). Sa structure peut être en feutre, en silicone ou, plus généralement, de même nature que la bande 140a déjà décrite.

Le diamètre (ou la largeur) d1 de ce patin est plus grand(e) de quelques millimètres ou centimètres que la largeur d2 de l'agrafe 361, de manière à remplacer alors la bande, avec dans ce cas un recouvrement extérieur plus localisé du conduit concerné.

Pour la fixation de l'endoprothèse de la figure 1, il est conseillé de procéder comme suit :

Pour la mise en place de l'implant 100, on peut tout d'abord procéder comme pour l'implant 100', de même pour la pose collier 140a (voir pose collier 140'a). Si le collier est comme celui de la figure 11, avec une lanière étanche au sang qui se prolonge en 365, 367 au-delà d'une zone centrale présentant une âme de renforcement 369 en forme d'anneau fendu élastiquement déformable radialement, une mise à longueur du collier 140a est si nécessaire effectuée, les prolongements 365, 367 étant alors sécables (tissu, tel que DACRON®).

On procède de la même manière pour la pose et l'ajustement du collier "aval" 140b, sauf que l'implant a alors déjà été entièrement largué dans le vaisseau V, puisqu'il ne s'étend qu'à l'endroit de l'anévrisme 110.

Pour fixer ensuite ensemble chaque collier/le vaisseau/l'implant, en particulier par une série d'agrafes 361, le praticien peut utiliser une agrafeuse endoscopique telle que décrite dans US-A-5 246 156, US-A-5 333 772, US-A-5 470 010 ou encore correspondant au modèle ENDOPATH EMS® de la société ETHICON, CINCINNATI, OH, U.S.A., qui permet d'agrafer uniquement à partir d'un côté. Outre cela, et qu'elle soit endoscopique ou non, ce type d'agrafeuse unilatérale, repérée 370 à la figure 12, est chargée avec des agrafes ayant d'origine une forme en " 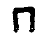 ", comme sur la figure 5, la manoeuvre de la détente 371, après avoir appliqué l'embout 373 de l'agrafeuse contre la surface extérieure du collier, ayant pour effet d'abord d'enfoncer une agrafe à travers les épaisseurs précitées (en lui maintenant sa forme d'origine sensiblement en " 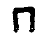 "), puis, en fin de course, de refermer ses extrémités pour parvenir à la forme en " 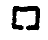 " recherchée et à la situation de la figure 9 où le collier, le vaisseau V et l'implant sont serrés ensemble. Entre les deux états, la longueur (ℓ₁ puis ℓ₂) de l'agrafe a donc été réduite au profit du serrage et de la fixation étanche.

A noter que cet effet de variation de longueur du moyen de fixation existerait également si l'on utilisait des rivets tels que décrits dans FR 97 16625, à la place d'agrafes.

Concernant l'étanchéité sanguine, l'utilisation d'un revêtement auto-obturant 372 (tel qu'une couche de silicone ; figure 11) sur chaque collier doit résoudre le problème à l'extérieur du vaisseau. Intérieurement, si un risque demeure le tampon intermédiaire 150 de la figure 9 apporte une solution. Mais il y a alors une épaisseur supplémentaire à faire traverser aux moyens de fixation transvasculaire. Une autre solution consiste à enduire extérieurement d'une couche de matière auto-obturante au moins les extrémités proximale et/ou distale du manchon de l'implant (voir zones 395 et 397 sur la figure 1).

Bien entendu, pour fixer ainsi de façon étanche les colliers, le praticien aura dû procéder à plusieurs séries d'incisions transcutanées, si une approche endoscopique a été préférée à une dénudation vasculaire.

Sur la figure 13, on a représenté une partie d'une doublure repérée ici 140b. Celle-ci a pour particularité que sa surface intérieure 355 (devant venir au contact du conduit V) est en matière "adhésive" ou favorisant l'adhésion au conduit, telle qu'une matière favorable à la croissance cellulaire, comme un polyester ou une mousse de TEFLON®. Par contre, sa surface extérieure 357 est en une manière "non-adhésive", ou plus exactement non favorable à l'adhésion sur une partie anatomique et en particulier non favorable à la croissance cellulaire à son contact, telle qu'une silicone. La doublure 140b peut comprendre deux couches 355, 357 comme sur la figure 13, avec par exemple une languette silicone extérieure et un tampon interne en polyester, ou une âme centrale (pour l'aspect mécanique) recouverte respectivement intérieurement et extérieurement de deux pellicules 355, 357. L'inverse peut être prévu sur la bande 150 ("adhésion" externe, face au conduit, et éventuellement "non adhésion" interne).

## Revendications

1. Ensemble médical pour le traitement d'une affection d'un conduit anatomique (V, V₁, V₂) comprenant :
- un implant (100, 100') introductible dans le conduit à traiter, l'implant ayant un axe général (100c) et présentant sensiblement selon cet axe deux extrémités libres, respectivement proximale et distale (100a, 100b ; 100'a, 100'b)
- au moins une doublure (140, 140a, 140b, 140'a) pour doubler extérieurement une partie au moins de la paroi du conduit, sensiblement en regard de l'extrémité proximale de l'implant,
- des moyens de fixation (321, 323, 361) de la doublure à l'implant, à travers la paroi du conduit,
- des moyens (310, 320, 370) de mise en place des moyens de fixation, lesquels sont distincts de l'implant, pour mettre en place ces moyens de fixation, de préférence depuis l'extérieur du conduit, en direction de l'implant, **caractérisé en ce que** la(les) doublure(s) (140, 140a, ...) présente(nt) une armature (303, 309, 369) pour être mécaniquement résistante à l'écrasement dans le sens de son(leur) épaisseur (e) afin de constituer un étai extérieur pour lesdits moyens de fixation traversant la paroi du conduit considéré.

2. Ensemble selon la revendication 1, **caractérisé en ce que** l'armature de la (des) doublure(s) (140, 140a, ...) est déformable pour se conformer à la forme extérieure du conduit considéré, comme un anneau fendu, sensiblement.

3. Ensemble selon l'une des revendications 1 et 2, **caractérisé en ce que** la(les) doublure(s) présente(nt) un coussinet de protection (305, 312), au moins d'un côté intérieur destiné à être placé contre le conduit considéré.

4. Ensemble selon l'une quelconque des revendications précédentes, **caractérisé en ce que** la(les) doublure(s) (140, 140a, ...) est(sont) sécable(s) pour être adaptée(s) à la section extérieure du conduit considéré.

5. Ensemble selon l'une quelconque des revendications précédentes, **caractérisé en ce que** les moyens de fixation consistant en des moyens de serrage (361) présentant, sensiblement parallèlement à leur axe d'introduction, une longueur (ℓ₁, ℓ₂) différente entre un premier état d'introduction à travers la doublure extérieure, le conduit et l'implant, et un deuxième état de fixation après introduction, pour serrer alors la paroi du conduit entre la doublure extérieure (140, 140a, ...) et l'implant intérieur (100, 100'), de manière à créer une étanchéité entre l'implant et la paroi dudit conduit vis-à-vis du fluide circulant dans le conduit.

6. Ensemble selon l'une quelconque des revendications précédentes, **caractérisé en ce que** la (certaines au moins des) doublure(s) (140"a) se présente(nt) comme un patin amortisseur localisé essentiellement à l'endroit des moyens de fixation, pour former écran à l'extérieur, et possiblement à l'intérieur, du conduit considéré entre ces moyens de fixation et ledit conduit.

7. Ensemble selon l'une quelconque des revendications précédentes, **caractérisé en ce que** les moyens de fixation comprennent des agrafes chirurgicales (361) et les moyens de mise en place comprennent une agrafeuse chirurgicale (370) adaptée pour introduire et recourber les extrémités libres (363) d'une agrafe depuis exclusivement un côté du conduit considéré.

8. Ensemble selon la revendication 7, **caractérisé en ce que** l'agrafeuse est une agrafeuse endoscopique (370) adaptée pour être approchée du conduit, par l'extérieur de celui-ci.

9. Ensemble selon la revendication 7 ou 8, **caractérisé en ce que** les agrafes (361) présentent un premier état originel d'introduction en forme sensiblement de "U", avant déformation par l'agrafeuse (370), pour pénétrer à travers la doublure, le conduit et l'implant, puis un second état de fixation en forme sensiblement de zone de pliure (363) des extrémités libres après déformation par l'agrafeuse lorsqu'elles ont pénétré la doublure, le conduit et l'implant.

10. Ensemble selon l'une quelconque des revendications précédentes, **caractérisé en ce que** la(chaque) doublure (140a, 140b ; 140'a) présente intérieurement une couche (355) en une matière favorisant la croissance cellulaire au contact du conduit.

## Patentansprüche

1. Medizinische Vorrichtung zur Behandlung einer Erkrankung eines anatomischen Ganges (V, V₁, V₂) mit:
- einem Implantat (100, 100'), das in den zu behandelnden Gang einführbar ist, eine allgemeine Achse (100c) hat und im wesentlichen entsprechend dieser Achse zwei freie Enden proximal bzw. distal (100a, 100b; 100'a, 100'b) aufweist,
- mindestens einer Verstärkung (140, 140a, 140b, 140'a), um außen mindestens einen Teil der Wand des Ganges im wesentlichen gegenüber dem proximalen Ende des Implantats zu verstärken,
- Befestigungsmitteln (321, 323, 361) der Verstärkung an dem Implantat quer durch die Wand des Ganges,
- Mitteln (310, 320, 370) zum Anordnen der Befestigungsmittel, die sich von dem Implantat unterscheiden, um diese Befestigungsmittel vorzugsweise von außerhalb des Ganges in Richtung des Implantates anzuordnen,
**dadurch gekennzeichnet, daß** die Verstärkung(en) (140, 140a, ...) eine Armierung (303, 309, 369) aufweist (aufweisen),um mechanisch gegen das Zusammendrücken in der Richtung ihrer Dicke (e) mechanisch widerstandsfähig zu sein, um eine äußere Stütze für die Befestigungsmittel zu bilden, welche die Wand des betrachteten Ganges durchqueren.

2. Vorrichtung nach Anspruch 1, **dadurch gekennzeichnet, daß** die Armierung der Verstärkung(en) (140, 140a, ...) verformbar ist (sind), um sich der äußeren Form des betrachteten Ganges im wesentlichen als ein geschlitzter Ring anzupassen.

3. Vorrichtung nach einem der Ansprüche 1 und 2, **dadurch gekennzeichnet, daß** die Verstärkung(en) ein Schutzfutter (305, 312) mindestens auf einer inneren Seite aufweist (aufweisen), das dazu bestimmt ist, gegen den betrachteten Gang angeordnet zu werden.

4. Vorrichtung nach einem der vorhergehenden Ansprüche, **dadurch gekennzeichnet, daß** die Verstärkung(en) (140, 140a, ....) schneidbar ist (sind), um dem äußeren Querschnitt des betrachteten Ganges angepaßt zu werden.

5. Vorrichtung nach einem der vorhergehenden Ansprüche, **dadurch gekennzeichnet, daß** die Befestigungsmittel aus Klemmitteln (361) bestehen, die im wesentlichen parallel zu ihrer Einführachse eine Länge (I₁, I₂) aufweisen, die unterschiedlich ist zwischen einem ersten Einführzustand quer durch die äußere Verstärkung, den Gang und das Implantat und einem zweiten Befestigungszustand nach dem Einführen, um dann die Wand des Ganges zwischen der äußeren Verstärkung (140, 140a, ...) und dem inneren Implantat (100, 100') derart zu klemmen, daß eine Dichtigkeit zwischen dem Implantat und der Wand des Ganges gegenüber dem in dem Gang zirkulierenden Fluid geschaffen wird.

6. Vorrichtung nach einem der vorhergehenden Ansprüche, **dadurch gekennzeichnet, daß** die (mindestens einige der) Verstärkung(en) (140"a) sich als ein Dämpfungsschuh darstellt (darstellen), der im wesentlichen an der Stelle der Befestigungsmittel angeordnet ist, um nach außen und nach Möglichkeit nach innen des betrachteten Ganges zwischen diesen Befestigungsmitteln und dem Gang einen Schirm zu bilden.

7. Vorrichtung nach einem der vorhergehenden Ansprüche, **dadurch gekennzeichnet, daß** die Befestigungsmittel chirurgische Klammern (361) und die Anordnungsmittel einen chirurgischen Heftapparat (370) aufweisen, der geeignet ist, die freien Enden (363) einer Klammer von ausschließlich einer Seite des betrachteten Ganges einzuführen und umzubiegen.

8. Vorrichtung nach Anspruch 7,**dadurch gekennzeichnet, daß** der Heftapparat ein endoskopischer Heftapparat (370) ist, der geeignet ist, dem Gang von außerhalb desselben genähert zu werden.

9. Vorrichtung nach Anspruch 7 oder 8,**dadurch gekennzeichnet, daß** die Klammern (361) einen ersten ursprünglichen Einführzustand in der Form im wesentlichen eines "U" vor der Verformung durch den Heftapparat (370) zum Durchdringen quer durch die Verstärkung, den Gang und das Implantat, aufweisen, dann einen zweiten Befestigungszustand in der Form im wesentlichen der Falzzone (363) der freien Enden nach Verformung durch den Heftapparat, wenn sie die Verstärkung, den Gang und das Implantat durchdrungen haben.

10. Vorrichtung nach einem der vorhergehenden Ansprüche, **dadurch gekennzeichnet, daß** die (jede) Verstärkung (140a, 140b; 140'a) innen eine Schicht (355) aus einem Material aufweist, welches das Zellwachstum in Kontakt mit dem Gang begünstigt.

## Claims

1. A medical set for treating an affection of an anatomical duct (V, V₁, V₂) comprising:
- an implant (100, 100') adapted to be inserted in the duct to be treated, the implant having a general axis (100c) and having substantially according to this axis two free ends, respectively proximal and distal (100a, 100b; 100'a, 100'b),
- at least one lining (140, 140a, 140b, 140'a) for externally lining at least a part of the duct wall, substantially opposite the proximal end of the implant,
- fastening means (321, 323, 361) for fastening the lining to the implant, through the wall of the duct,
- set up means (310, 320, 370) for the fastening means, said fastening means being distinct from the implant, for setting up these fastening means, preferably from the outside of the duct, in the direction of the implant, **characterized in that** the lining(s) (140, 140a,...) has (have) a frame (303, 309, 369) for being thicknesswise mechanically resistant to crushing in order to form an external prop for said fastening means crossing the wall of said duct.

2. A set according to claim 1, **characterized in that** the frame of the lining(s) (140, 140a,...) can be deformed in order to adapt to the external shape of said duct, like a slit ring, substantially.

3. A set according to anyone of claim 1 and 2, **characterized in that** the lining(s) has(have) on at least an internal side a protective pad (305, 312) to be set against said duct.

4. A set according to anyone of preceding claims, **characterized in that** the lining(s) (140, 140a,...) is(are) divisible in order to be adapted to the external section of said duct.

5. A set according to anyone of preceding claims, **characterized in that** the fastening means are made of clamping means (361) having, substantially parallel to their introduction axis, a length (ℓ₁, ℓ₂) different between a first state of introduction through the external lining, the duct and the implant, and a second state of fixation after introduction, for then clamping the duct wall between the external lining (140, 140a, ...) and the internal implant (100, 100'), in order to create a tightness between the implant and the wall of said duct in relation to the fluid circulating in the duct.

6. A set according to anyone of preceding claims, **characterized in that** the lining (at least some of the lining) (140"a) is(are) like an absorbing pad mainly situated at the fastening means location, for forming a shield external to, and possibly internal to, said duct, between these fastening means and said duct.

7. A set according to anyone of preceding claims, **characterized in that** the fastening means comprise chirurgical staples (361) and the setting means comprise a chirurgical stapler (370) adapted for introducing and bend back the free ends (363) of a staple from exclusively one side of said duct.

8. A set according to claim 7, **characterized in that** the stapler is an endoscopic stapler (370) adapted to be brought near the duct, from the exterior of it.

9. A set according to claim 7 or 8, **characterized in that** the staples (361) have an introduction primary first state with ansubstantially "U" shape, before deformation by the stapler (370) in order to penetrate through the lining, the duct and the implant, then a second fixation state with a shape having substantially a zone of folding (363) of the free ends after deformation by the stapler when they have penetrated the lining, the duct and the implant.

10. A set according to anyone of preceding claims **characterized in that** each lining (140a, 140b; 140'a) has an internal coating (355) made of a material favouring the cellular growth when in contact with the duct.
